# EUROPÄISCHE PATENTSCHRIFT

(11) **EP 1 325 736 B1**
(45) Veröffentlichungstag und Bekanntmachung des Hinweises auf die Patenterteilung: **11.08.2004**
(21) Anmeldenummer: 02028820.5
(22) Anmeldetag: 21.12.2002
(51) Int. Cl.: A61K 7/46, A61K 9/107, A61K 47/34

(54) **Kosmetische oder pharmazeutische Öl-in-Wasser-Emulsionen, die ein oder mehrere hydrophob modifizierte copolymere Polyglutaminsäurederivate enthalten**
Cosmetic or pharmaceutical oil-in-water emulsions comprising one or more hydrophobically modified copolymeric polyglutamic acid derivatives
Compositions cosmétiques ou pharmaceutiques sous forme d'émulsion huile-dans-eau contenant un ou plusieurs dérivés copolymères d'acide polyglutamique modifiés de manière hydrophobe

(30) Priorität: 04.01.2002 DE 10200209
(43) Veröffentlichungstag der Anmeldung: 09.07.2003
(73) Patentinhaber: Goldschmidt AG, 45127 Essen (DE)
(72) Erfinder: Dietz, Thomas, Dr., 45127 Essen (DE); Hameyer, Peter, 45138 Essen (DE); Schick, Ute, Dr., 50676 Köln (DE)

(56) Entgegenhaltungen:
- DE-A- 19 822 601
- US-A- 5 852 109

## Beschreibung

Die Erfindung betrifft die Verwendung von hydrophob modifizierten copolymeren Polyglutaminsäurederivaten definierter Struktur zur Herstellung kosmetischer oder pharmazeutischer Öl-in-Wasser-Emulsionen sowie Öl-in-Wasser-Emulsionen, die diese enthalten.

Hydrophob modifizierte Polyaminosäurederivate sind bereits seit langem bekannt, ebenso deren Verwendung zur Herstellung kosmetischer oder pharmazeutischer Emulsionen. So beansprucht z. B. die DE-A-22 53 190 die Verwendung von Polyasparaginsäureamiden als Tenside bzw. grenzflächenaktive Verbindungen, insbesondere Waschmittel und Kosmetika, die diese Polyasparaginsäureamide enthalten. In dieser Schrift wird auch eine Hautcreme als Beispiel angeführt, die 25 % eines erfindungsgemäßen Polyasparaginsäureamids enthält.

In der DE-A-195 24 097 werden kosmetische Mittel beansprucht, die neben Alkyl- und/oder Alkenyloligoglykosiden und/oder Fettsäure-N-alkylpolyhydroxyalkylamiden auch hydrophobierte Oligopeptide enthalten. Eine Gruppe von hydrophobierten Oligopeptiden stellen Umsetzungsprodukte von Polyasparaginsäuren mit Aminen mit 1 bis 22 Kohlenstoffatomen dar. Der Zusatz der hydrophobierten Oligopeptide zu den ausgewählten Zuckertensiden führt zur Einstellung einer vorteilhaft hohen Viskosität, einer synergistischen Verbesserung des Basisschaums und der Schaumbeständigkeit. Zudem vermitteln die erfindungsgemäßen Mittel ein angenehmes Hautgefühl.

In der DE-A-195 45 678 werden copolymere Polyaminosäureester und deren Verwendung unter anderem als Emulgatoren in kosmetischen Präparaten beschrieben. Es werden als Beispiele einfach aufgebaute O/W- bzw. W/O-Emulsionen, bestehend aus Wasser, Emulgator und Öl, angeführt, die mindestens 3 Wochen stabil waren.

In der DE-A-197 20 771 wird ein Verfahren zur Herstellung von Polyasparaginsäureamiden sowie deren Verwendung zur Herstellung von kosmetischen und/oder pharmazeutischen Zubereitungen beansprucht. Diese Zubereitungen können z. B. Haarshampoos, Haarlotionen, Schaumbäder, Cremes, Lotionen oder Salben sein.

Die EP-A-958 811 beansprucht kosmetische O/W-Emulsionen, enthaltend ein oder mehrere hydrophob modifizierte Polyasparaginsäurederivate, einen oder mehrere Konsistenzgeber, sowie gegebenenfalls weitere Coemulgatoren sowie übliche Hilfs- und Zusatzstoffe. Diese O/W-Emulsionen können als Hautpflegemittel, Tagescreme, Nachtcreme, Pflegecreme, Nährcreme, Bodylotion, pharmazeutische Salbe und Lotion, Aftershave Lotion und Sonnenschutzmittel verwendet werden.

Zur Herstellung von Emulsionen werden Emulgatoren benötigt. Emulgatoren sind Tenside mit mindestens einem hydrophilen und mindestens einem hydrophoben bzw. lipophilen Teil. Der hydrophobe Teil bzw. lipophile Teil wird durch gesättigte oder ungesättigte, lineare oder verzweigte Alkylreste mit überwiegend 12 bis 22 Kohlenstoffatomen, durch Polypropylenglykol oder Polydimethylsiloxan gebildet. Die Emulgatoren können nach der chemischen Struktur des hydrophilen Teils in nichtionogene, anionaktive, kationaktive oder amphotere Emulgatoren eingeteilt werden. Beispiele für anionaktive hydrophile Gruppen sind neutralisierte Carboxyl-, Sulfat- oder Phosphatgruppen. Beispiele für anionaktive O/W-Emulgatoren sind selbstemulgierendes Glycerylstearat, das durch partielle Verseifung von Glycerinmonodistearat mit Kaliumhydroxid erhalten wird, wobei das hierbei gebildete Kaliumstearat (Seife) als emulgieraktive Komponente wirkt. Weitere Beispiele für anionaktive O/W-Emulgatoren sind Aminseifen wie Triethanolammoniumstearat, Natriumcetearylsulfat, Kaliumcetylphosphat, Natriumglycerylstearatcitrat und Natriumstearoyllactylat. Ein besonderer Vorteil von anionaktiven Emulgatoren ist ihre außerordentlich hohe Emulgierfähigkeit. Ein entscheidender Nachteil ist jedoch die Einschränkung des pH-Wert-Bereichs damit hergestellter Emulsionen. Mit selbstemulgierendem Glycerylstearat lassen sich zum Beispiel nur Emulsionen mit einem pH-Wert von > 6,8 herstellen, da bei niedrigeren pH-Werten die emulgieraktive Komponente unwirksam ist. Es sind jedoch kosmetische Öl-in-Wasser-Emulsionen gewünscht, deren pH-Wert dem natürlichen pH-Wert der Haut entspricht, d. h. bei ca. 5,5 liegt. Die Aufgabe bestand nun darin, anionaktive Emulgatoren bereitzustellen, mit denen kosmetische oder pharmazeutische Öl-in-Wasser-Emulsionen mit hautfreundlichem pH-Wert von 5,5 hergestellt werden können, die sich gleichzeitig durch gute Wärme- und Kältestabilität, durch ein brillantes Aussehen sowie durch ein angenehmes Hautgefühl auszeichnen. Ferner sollten die Emulgatoren auf nachwachsenden Rohstoffen basieren, keine oxidationsempfindlichen Reste wie z. B. Polyethylenglykolreste enthalten und biologisch abbaubar sein.

Es wurde nun überraschend gefunden, dass mit Hilfe von hydrophob modifizierten copolymeren Polyglutaminsäurederivaten bestimmter Zusammensetzung Öl-in-Wasser-Emulsionen mit einem sehr feinem Dispersiongrad, gleichbedeutend einem brillanten Aussehen, mit sehr guter Kälte- und Wärmestabilität hergestellt werden können, deren pH-Wert auf den des natürlichen pH-Wertes der Haut eingestellt werden kann. Diese Emulgatoren basieren auf pflanzlichen Rohstoffen, sind nicht oxidationsempfindlich und biologisch abbaubar. Es wurde ferner gefunden, dass sie bereits in sehr geringer Konzentration von < 1 % hochwirksame Öl-in-Wasser-Emulgatoren sind, wenn man sie vorzugsweise mit wachsartigen Konsistenzgebern ("polare Wachse" bzw. "hydrophile Wachse") wie Glycerylmono-distearat, Stearylalkohol, Cetylalkohol oder Stearinsäure kombiniert.

Ein Gegenstand der Erfindung sind daher kosmetische oder pharmazeutische Öl-in-Wasser-Emulsionen, die ein oder mehrere hydrophob modifizierte copolymere Polyglutaminsäurederivate und weitere Hilfs- und Zusatzstoffe enthalten, bei welchen als copolymere Polyglutaminsäurederivate Verbindungen verwendet werden, welche durch gleichzeitige oder stufenweise Umsetzung von Glutaminsäure und mindestens einer weiteren α-Aminosäure und/oder deren Derivaten und Aminen in Abwesenheit von Lösungsmitteln und Katalysatoren, nach an sich bekannten Verfahren hergestellt werden.

Ein weiterer Gegenstand der Erfindung sind kosmetische oder pharmazeutische Öl-in-Wasser-Emulsionen, welche dadurch gekennzeichnet sind, dass sie enthalten
a) ein oder mehrere hydrophob modifizierte copolymere Polyglutaminsäurederivate, hergestellt gemäß Anspruch 1 und
b) ein oder mehrere polare Wachse, ausgewählt aus der Gruppe bestehend aus Fettalkoholen mit 12 bis 22 Kohlenstoffatomen, bestehend aus Fettsäuren mit 12 bis 22 Kohlenstoffatomen und/oder bestehend aus Glycerin- oder Polyglycerinpartialestern von Fettsäuren mit 12 bis 22 Kohlenstoffatomen und
c) ein oder mehrere kosmetische Öle.

Ein weiterer Gegenstand der Erfindung sind kosmetische oder pharmazeutische Öl-in-Wasser-Emulsionen gemäß Anspruch 2, welche dadurch gekennzeichnet sind, dass der Anteil der Komponente (a) zwischen 0,1 und 2,0 %, der Anteil der Komponente (b) 0,5 bis 8,0 % und der Anteil der Komponente (c) 1,0 bis 60 % an der Gesamtemulsion beträgt.

Weitere Gegenstände der Erfindung sind gekennzeichnet durch die Ansprüche.

Die erfindungsgemäß mitverwendeten Copolymeren bestehen zu 30 bis 90 Gew.-% der vorhandenen Einheiten aus Struktureinheiten der allgemeinen Formel (I) und zu 70 bis 30 Gew.-% der allgemeinen Formel (II) 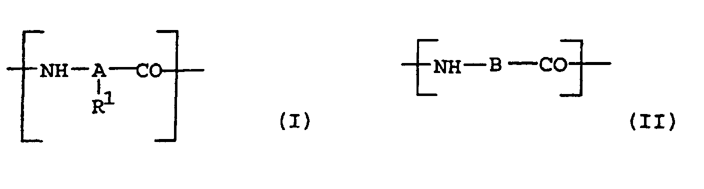 worin
- A: ein trifunktionelles Radikal mit drei C-Atomen und den Strukturen ist 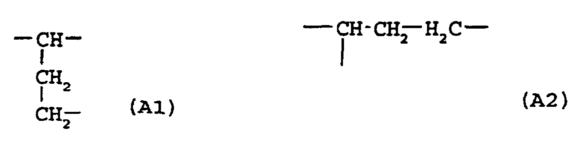 und in denen
- R¹: die Bedeutung von R², R³ und R⁴ haben kann,
wobei
- R²: gleiche oder verschiedene, Amidreste -C(O)NH-R⁹ mit
R⁹ geradkettige oder verzweigte, gesättigte und ungesättigte Alkylreste mit 1 bis 24, vorzugsweise 6 bis 24 C-Atomen oder Radikale der Struktur - C(O)NH-X-R⁹ sind, wobei
X eine Oligo- oder Polyoxyalkylenkette mit 1 bis 100 Oxyalkyleneinheiten, vorzugsweise Ethlenoxydeinheiten, ist,
- R³: gleiche oder verschiedene, Amidreste -C(O)NH-(CH₂)n-N(R¹⁰)(R¹¹) mit
n 2 bis 10, vorzugsweise 2 bis 4 und
R¹⁰, R¹¹ unabhängig voneinander geradkettige oder verzweigte, gesättigte oder ungesättigte Alkylreste mit 1 bis 24 C-Atomen und/oder Hydroxyalkylreste sind
- R⁴: die Bedeutung COO⁻X⁺, mit
X⁺ ein oder mehrere Reste aus der Gruppe der Alkali-, Erdalkalimetalle, Wasserstoff oder Ammonium, [NR⁵R⁶R⁷R⁸] ⁺, hat, worin
R⁵ bis R⁸ unabhängig voneinander Wasserstoff, Alkyl oder Hydroxyalkyl oder [NH₃-X-R⁹] ⁺, und/oder [NH₃-R⁹] ⁺, und/oder [NH₃-(CH₂)ₙ-N(R¹⁰)(R¹¹)] ⁺, bedeuten,
und mindestens jeweils ein Rest R¹ die Bedeutung von R², R³ und/oder R⁴ annehmen muss und die Einheiten [-NH-B-CO-] Bausteine aus der Gruppe der proteinogenen und/oder nicht proteinogenen Aminosäuren (H₂N-B-COOH) sind, worin B der Rest der jeweiligen Aminosäure ist.

Als Aminosäurebausteine [-NH-B-CO] aus der Gruppe der proteinogenen Aminosäuren H₂N-B-COOH kommen z. B. Glycin, Alanin, Leucin, Isoleucin, Phenylalanin, Tyrosin, Serin, Cystein, Methionin, Glutaminsäure, Glutamin, Asparaginsäure, Asparagin, Lysin, Hydroxylysin, Arginin, Tryptophan, Histidin, Valin, Threonin, Prolin, Hydroxyprolin sowie deren Derivate in Frage; nicht proteinogene Aminosäuren können z. B. β-Alanin, ω-Amino-1-alkansäuren etc. sein.

Als erfindungsgemäß mitverwendbare Amine zur Herstellung der Copolymeren kommen Verbindungen in Betracht, die mindestens eine mit Carboxylgruppen reaktionsfähige Aminogruppe enthalten, wie beispielsweise:
NH₂-R⁹ und/oder H₂N-X-R⁹ und/oder H₂N-(CH₂)ₙ-N(R¹⁰)(R¹¹) worin R⁹, R¹⁰, R¹¹, X und n die oben angegebene Bedeutung haben.

Vorzugsweise werden solche Verbindungen eingesetzt, welche bei den gegebenen Reaktionsbedingungen flüssig sind und nicht aus dem Reaktionsgemisch abdestillieren. Das sind die höheren Amine mit 6 oder mehr C-Atomen in der Alkylkette wie die homologe Reihe der Fettamine H₂N-R⁹.

Diese Fettamine werden nach bekannten Verfahren durch Umsetzung von Fettsäuren mit NH₃ in Gegenwart von Katalysatoren zum Nitril und anschließender Hydrierung zum primären oder sekundären Amin hergestellt.

Als Fettsäuren werden hierzu einzeln oder in Mischungen Säuren wie Caprylsäure, Caprinsäure, 2-Ethyl-hexansäure, Laurinsäure, Myristinsäure, Palmitinsäure, Palmitoleinsäure, Isostearinsäure, Stearinsäure, Hydroxystearinsäure (Ricinolsäure), Dihydroxystearinsäure, Ölsäure, Linolsäure, Petroselinsäure, Elaidinsäure, Arachinsäure, Behensäure und Erucasäure, Gadoleinsäure sowie die bei der Druckspaltung natürlicher Fette und Öle anfallenden technischen Mischungen wie Ölsäure, Linolsäure, Linolensäure, und insbesondere Rapsölfettsäure, Sojaölfettsäure, Sonnenblumenölfettsäure, Tallölfettsäure mitverwendet. Geeignet sind prinzipiell alle Fettsäuren mit ähnlicher Kettenverteilung.

Der Gehalt dieser Fettsäuren bzw. Fettsäureester an ungesättigten Anteilen, wird - soweit dies erforderlich ist - durch die bekannten katalytischen Hydrierverfahren auf eine gewünschte Jodzahl eingestellt oder durch Abmischung von vollhydrierten mit nichthydrierten Fettkomponenten erzielt. Die Jodzahl, als Maßzahl für den durchschnittlichen Sättigungsgrad einer Fettsäure, ist die Jodmenge, welche von 100 g der Verbindung zur Absättigung der Doppelbindungen aufgenommen wird.

Vorzugsweise werden teilgehärtete C₈-C₁₈-Kokos- bzw. Palmfettsäuren, Rapsölfettsäuren, Sonnenblumenölfettsäuren Sojaölfettsäuren und Tallölfettsäuren, mit Jodzahlen im Bereich von ca. 80 bis 150 und insbesondere technische C₈-C₁₈-Kokosfettsäuren eingesetzt, wobei gegebenenfalls eine Auswahl von cis-/trans-Isomeren wie elaidinsäurereiche C₁₆-C₁₈-Fettsäureschnitte von Vorteil sein können. Sie sind handelsübliche Produkte und werden von verschiedenen Firmen unter deren jeweiligen Handelsnamen angeboten.

Weitere mitverwendbare Amine sind solche der allgemeinen Formel H₂N- (CH₂)ₙ-N(R¹⁰) (R¹¹), welche neben einer primären Aminogruppe noch mindestens eine sekundäre oder vorzugsweise tertiäre Aminogruppe enthalten wie insbesondere Dimethylaminopropylamin (DMAPA).

Weitere mitverwendbare Amine sind solche der allgemeinen Formel H₂N-X-R⁹ wie insbesondere die von der Firma Huntsman unter dem Markennamen Jeffamine® vertriebenen Verbindungen.

Weitere mitverwendbare Amine sind Imidazolinringe enthaltende Verbindungen wie beispielsweise die aus den oben angeführten Fettsäuren und Diethylentriamin die nach bekannten Methoden hergestellt werden.

In den erfindungsgemäß mitverwendeten Copolymeren ist das Verhältnis Glutaminsäure/Coaminosäuren: 90/10 bis 30/70 Gew.-% vorzugsweise 90/10 bis 50/50 Gew.-% und das Verhältnis Aminosäuren/Amin: 90/10 bis 30/70 Gew.-%, vorzugsweise 40/60 bis 60/40.

Die erfindungsgemäß mitverwendeten Copolymeren werden durch gleichzeitige oder stufenweise Umsetzung von Glutaminsäure und mindestens einer weiteren α-Aminosäure und/oder deren Derivaten und Aminen in Abwesenheit von Lösungsmitteln und Katalysatoren, unter Kondensationsbedingungen hergestellt.

Ein Verfahren zur Herstellung der copolymeren Polypeptide besteht darin, dass in erster Stufe Glutaminsäure und/oder deren Derivate in Abwesenheit von Lösungsmitteln und Katalysatoren, unter Kondensationsbedingungen mit Aminen, gegebenenfalls bei subatmosphärischem Druck und Entfernung des Kondensats aus der Reaktionsmischung, umgesetzt werden und dass in zweiter Stufe eine oder mehrere weitere Aminosäuren und/oder deren Derivaten und/oder ein oder mehrere gleiche und/oder weitere Amine gleichzeitig oder in beliebiger Reihenfolge zugesetzt und in Abwesenheit von Lösungsmitteln und Katalysatoren, unter Kondensationsbedingungen, gegebenenfalls bei subatmosphärischem Druck und Entfernung des Kondensats aus der Reaktionsmischung, umgesetzt werden.

Ein weiteres Verfahren der Herstellung der copolymeren Polypeptide besteht darin, dass in erster Stufe Glutaminsäure und/oder deren Derivate mit einer oder mehreren weiteren α-Aminosäuren und/oder deren Derivaten, in Abwesenheit von Lösungsmitteln und Katalysatoren, unter Kondensationsbedingungen, gegebenenfalls bei subatmosphärischem Druck und Entfernung des Kondensats aus der Reaktionsmischung, umgesetzt werden und dass in zweiter Stufe ein oder mehrere Amine gleichzeitig oder in beliebiger Reihenfolge in Abwesenheit von Lösungsmitteln und Katalysatoren, unter Kondensationsbedingungen, gegebenenfalls bei subatmosphärischem Druck und Entfernung des Kondensats aus der Reaktionsmischung, mit dem Reaktionsprodukt der ersten Stufe umgesetzt werden.

Ein weiteres Verfahren der Herstellung der copolymeren Polypeptide besteht darin, dass in erster Stufe Glutaminsäure und/oder deren Derivate mit einer oder mehreren weiteren α-Aminosäuren und/oder deren Derivaten, mit einem oder mehreren Aminen gleichzeitig oder in beliebiger Reihenfolge in Abwesenheit von Lösungsmitteln und Katalysatoren, unter Kondensationsbedingungen, gegebenenfalls bei subatmosphärischem Druck und Entfernung des Kondensats aus der Reaktionsmischung, umgesetzt werden und dass in zweiter Stufe Glutaminsäure und/oder eine oder mehrere weitere Coaminosäuren und/oder deren Derivate und/oder ein oder mehrere gleiche und/oder weitere Amine gleichzeitig oder in beliebiger Reihenfolge in Abwesenheit von Lösungsmitteln und Katalysatoren, unter Kondensationsbedingungen, gegebenenfalls bei subatmosphärischem Druck und Entfernung des Kondensats aus der Reaktionsmischung, mit dem Reaktionsprodukt der ersten Stufe umgesetzt werden.

Ein weiteres Verfahren der Herstellung der copolymeren Polypeptide besteht darin, dass in erster Stufe mindestens eine Aminosäure und/oder deren Derivate mit gegebenenfalls Aminen in einem Verhältnis gemischt werden, dass die Mischungen bei Kondensationstemperatur flüssig sind und in Abwesenheit von Lösungsmitteln und Katalysatoren auf mindestens Schmelztemperatur aufgeheizt werden und in zweiter Stufe gegebenenfalls weitere Coaminosäuren und/oder Amine gleichzeitig oder in beliebiger Reihenfolge zudosiert werden und unter Kondensationsbedingungen, gegebenenfalls bei subatmosphärischem Druck und unter Entfernung des Kondensats aus der Reaktionsmischung, mit der Mischung oder dem Reaktionsprodukt der ersten Stufe umgesetzt werden.

Zusätzlich zu den erfindungsgemäß verwendeten hydrophob modifizierten Polyglutaminsäurederivaten können noch andere in der Kosmetik übliche Emulgatoren verwendet werden. Als weitere Emulgatoren kommen beispielsweise nichtionogene Tenside aus mindestens einer der folgenden Gruppen in Frage:
- Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe
- C₁₂-C₁₈-Fettsäuremono- und -diester von Anlagerungsprodukten von 1 bis 30 Mol Ethylenoxid an Glycerin
- Glycerinmono- und -diester und Sorbitanmono- und -diester von gesättigten und ungesättigten Fettsäuren mit 6 bis 22 Kohlenstoffatomen und deren Ethylenoxidanlagerungsprodukte
- Alkylmono- und -oligoglycoside mit 8 bis 22 Kohlenstoffatomen im Alkylrest und deren ethoxylierte Analoga
- Anlagerungsprodukte von 15 bis 60 Mol Ethylenoxid an Ricinusöl und/oder gehärtetes Ricinusöl
- Polyol- und insbesondere Polyglycerinester, wie beispielsweise Polyglycerinpolyricinoleat, Polyglycerin-12-hydroxystearat oder Polyglycerindimerat. Ebenfalls geeignet sind Gemische von Verbindungen aus mehreren dieser Substanzklassen
- Anlagerungsprodukte von 2 bis 15 Mol Ethylenoxid an Ricinusöl und/oder gehärtetes Ricinusöl
- Partialester auf Basis linearer, verzweigter, ungesättigter bzw. gesättigter C₆-C₂₂-Fettsäuren, Ricinolsäure sowie 12-Hydroxystearinsäure und Glycerin, Polyglycerin, Pentaerythrit, Dipentaerythrit, Zuckeralkohole (beispielsweise Sorbit), Alkylglucoside (beispielsweise Methylglucosid, Butylglucosid, Laurylglucosid) sowie Polyglucoside (beispielsweise Cellulose)
- Mono-, Di- und Trialkylphosphate sowie Mono-, Di- und/oder Tri-PEG-alkylphosphate und deren Salze
- Wollwachsalkohole
- Polysiloxan-Polyalkyl-Polyether-Copolymere beziehungsweise entsprechende Derivate
- Mischester aus Pentaerythrit, Fettsäuren, Citronensäure und Fettalkohol gemäß DE-B-11 65 574 und/oder Mischester von Fettsäuren mit 6 bis 22 Kohlenstoffatomen, Methylglucose und Polyolen, vorzugsweise Glycerin oder Polyglycerin sowie
- Polyalkylenglycole
- Betaine
- Esterquats
- Natrium-, Kalium- oder Ammoniumsalze von langkettigen Alkylund Alkylethersulfonsäuren

Die Anlagerungsprodukte von Ethylenoxid und/oder von Propylenoxid an Fettalkohole, Fettsäuren, Alkylphenole, Glycerinmono- und diester sowie Sorbitanmono- und -diester von Fettsäuren oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/oder Propylenoxid und Substrat, mit denen die Anlagerungsreaktion durchgeführt wird, entspricht.

Weiterhin können als Emulgatoren zwitterionische Tenside verwendet werden. Als zwitterionische Tenside werden solche oberflächenaktiven Verbindungen bezeichnet, die im Molekül mindestens eine quartäre Ammoniumgruppe und mindestens eine Carboxylat- und eine Sulfonatgruppe tragen. Besonders geeignete zwitterionische Tenside sind die sogenannten Betaine wie die N-Alkyl-N,N-dimethylammoniumglycinate, beispielsweise das Cocosalkyldimethylammoniumglycinat, N-Acylaminopropyl-N,N-dimethylammoniumglycinate, beispielsweise das Cocosacylaminopropyldimethylammoniumglycinat, und 2-Alkyl-3-carboxylmethyl-3-hydroxyehtylimidazoline mit jeweils 8 bis 18 C-Atomen in der Alkyl- oder Acylgruppe sowie das Cocosacylaminoethylhydroxyethylcarboxymethylglycinat. Besonders bevorzugt ist das unter der CTFA-Bezeichnung Cocamidopropyl Betaine bekannte Fettsäureamid-Derivat. Ebenfalls geeignete Emulgatoren sind ampholytische Tenside. Unter ampholytischen Tensiden werden solche oberflächenaktiven Verbindungen verstanden, die außer einer C₈-C₁₈-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Cocosalkylaminopropionat, das Cocosacylaminoethylaminopropionat und das C₁₂-C₁₈-Acylsarcosin. Neben den ampholytischen kommen auch quartäre Emulgatoren in Betracht, wobei solche vom Typ der Esterquats, vorzugsweise methylquaternierte Difettsäuretriethanolaminester-Salze, besonders bevorzugt sind.

Als Konsistenzgeber (hydrophile bzw. polare Wachse) kommen in erster Linie Fettalkohole oder Hydroxyfettalkohole mit 12 bis 22 und vorzugsweise 16 bis 18 Kohlenstoffatomen und daneben Partialglyceride, Fettsäuren oder Hydroxyfettsäuren in Betracht. Geeignete Verdickungsmittel (Hydrocolloide) sind beispielsweise Polysaccharide, insbesondere Xanthan-Gum, Guar-Guar, Agar-Agar, Alginate und Tylosen, Carboxymethylcellulose und Hydroxyethylcellulose, ferner höhermolekulare Polyethylenglycolmono- und diester von Fettsäuren, Polyacrylate (beispielsweise Carbopole von Goodrich, TEGO Carbomere von Goldschmidt oder Synthalene von Sigma), Polyacrylamide, Polyvinylalkohol und Polyvinylpyrrolidon, Tenside wie beispielsweise ethoxylierte Fettsäureglyceride, Ester von Fettsäuren mit Polyolen wie beispielsweise Pentaerythrit oder Trimethylolpropan, Fettalkoholethoxylate mit eingeengter Homologenverteilung oder Alkyloligoglucoside.

Als Ölphase kommen beispielsweise solche Ölkomponenten in Frage, die als kosmetische und pharmazeutische Ölkomponenten sowie als Gleit- und Schmiermittelkomponenten bekannt sind. Hierzu zählen insbesondere Mono- oder Diester der Kohlensäure (Carbonate) sowie von linearen und/oder verzweigten Mono- und/oder Dicarbonsäuren mit 2 bis 44 C-Atomen mit linearen und/oder verzweigten gesättigten oder ungesättigten Alkoholen mit 1 bis 22 C-Atomen. Ebenso sind im erfindungsgemäßen Sinne die Veresterungsprodukte aliphatischer, difunktioneller Alkohole mit 2 bis 36 C-Atomen mit monofunktionellen aliphatischen Carbonsäuren mit 1 bis 22 C-Atomen geeignet. Als Ölkomponenten geeignete Monoester sind beispielsweise die Methylester und Isopropylester von Fettsäuren mit 12 bis 22 C-Atomen wie beispielsweise Methyllaurat, Methylstearat, Methyloleat, Methylerucat, Isopropylpalmitat, Isopropylmyristat, Isopropylstearat, Isopropyloleat in Frage.

Andere geeignete Monoester sind beispielsweise n-Butylstearat, n-Hexyllaurat, n-Decyloleat, Isooctylstearat, Isononylpalmitat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat sowie Ester, die aus technischen aliphatischen Alkoholschnitten und technischen, aliphatischen Carbonsäuregemischen erhältlich sind, beispielsweise Ester aus ungesättigten Fettalkoholen mit 12 bis 22 C-Atomen und gesättigten und ungesättigten Fettsäuren mit 12 bis 22 C-Atomen, wie sie aus tierischen und pflanzlichen Fetten zugänglich sind. Geeignet sind aber auch natürlich vorkommende Monoester- bzw. Wachsester-Gemische, wie sie beispielsweise im Jojobaöl oder im Spermöl vorliegen.

Geeignete Diester sind beispielsweise Kohlensäurediester (Dialkylcarbonate) wie Di-(2-Ethylhexyl)-carbonat, Dicaprylylcarbonat, weitere geeignete Diester sind Dicarbonsäureester wie Di-n-butyladipat, Di-n-butyl-sebacat, Di-(2-ethylhexyl)-adipat, Di-(2-hexyldecyl)-succinat, Diisotridecylacelat. Geeignete Diolester sind beispielsweise Ethylenglycoldioleat, Ethylenglycol-diisotridecanoat, Propylenglycol-di-(2-ethylhexanoat), Butandioldi-isostearat und Neopentylglycol-di-caprylat.

Ebenso als Ölkomponente geeignet sind die Fettsäuretriglyceride, wobei unter diesen die natürlich vorkommenden Öle und Fette bevorzugt sind. So kommen beispielsweise natürliche, pflanzliche Öle, beispielsweise Olivenöl, Sonnenblumenöl, Sojaöl, Erdnußöl, Rapsöl, Mandelöl, Palmöl aber auch die flüssigen Anteile des Kokosöls oder des Palmkernöls sowie tierische Öle wie beispielsweise Klauenöl, die flüssigen Anteile des Rindertalgs oder auch synthetische Triglyceride von Capryl-Caprinsäure-Gemischen, Triglyceride aus technischer Ölsäure oder aus Palmitinsäure-Ölsäure-Gemischen als Ölkomponenten in Frage.

Als weitere Hilfs- und Zusatzstoffe kommen unter anderem UV-Lichtschutzfilter in Frage.

Unter UV-Lichtschutzfiltern sind organische Substanzen zu verstehen, die in der Lage sind, ultraviolette Strahlen zu absorbieren und die aufgenommene Energie in Form längerwelliger Strahlung, beispielsweise Wärme wieder abzugeben. UVB-Filter können öllöslich oder wasserlöslich sein. Als öllösliche Substanzen sind beispielsweise zu nennen:
- 3-Benzylidencampher und dessen Derivate, beispielsweise 3-(4-Methylbenzyliden)-camphor
- 4-Aminobenzoesäurederivate, vorzugsweise 4-(Dimethylamino)benzoesäure-2-ethylhexylester, 4-(Dimethylamino)benzoesäure-2-ethylhexylester und 4-(Dimethylamino)benzoesäureamylester
- Ester der Zimtsäure, vorzugsweise 4-Methoxyzimtsäure-2-ethylhexylester, 4-Methoxyzimtsäureisopentylester, 2-Cyan-3-phenyl-zimtsäure-2-ethylhexylester (Octocrylene)
- Ester der Salicylsäure, vorzugsweise Salicylsäure-2-ethylhexylester, Salicylsäure-4-isopropylbenzylester, Salicylsäurehomomenthylester
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon
- Ester der Benzalmalonsäure, vorzugsweise 4-Methoxybenzalmalonsäuredi-2-ethylhexylester
- Triazinderivate, wie beispielsweise 2,4,6-Trianilino-(p-carbo-2'-ethyl-1'-hexyloxy)-1,3,5-triazin und Octyltriazon.
- Propan-1,3-dione, wie beispielsweise 1-(4-tert.Butylphenyl)-3-(4'-methoxyphenyl)propan-1,3-dion.

Als wasserlösliche Substanzen kommen in Frage:
- 2-Phenylbenzimidazol-5-sulfonsäure und deren Alkali-, Erdalkali-, Ammonium-, Alkylammonium-, Alkanolammonium- und Glucammoniumsalze
- Sulfonsäurederivate von Benzophenon, vorzugsweise 2-Hydroxy-4-methoxybenzophenon-5-sulfonsäure und ihre Salze
- Sulfonsäurederivate des 3-Benzylidencamphers, wie beispielsweise 4-(2-Oxo-3-bornylidenmethyl)benzolsulfonsäure und 2-Methyl-5-(2-oxo-3-bornyliden)sulfonsäure und deren Salze.

Als typische UV-A-Filter kommen insbesondere Derivate des Benzoylmethans in Frage, wie beispielsweise 1-(4'-tert.Butylphenyl)-3-(4'-methoxyphenyl)propan-1,3-dion oder 1-Phenyl-3-(4'-isopropylphenyl)propan-1,3-dion. Die UV-A- und UV-B-Filter können selbstverständlich auch in Mischungen eingesetzt werden. Neben den genannten löslichen Stoffen kommen für diesen Zweck auch unlösliche Pigmente, nämlich feindisperse Metalloxide beziehungsweise Salze in Frage, wie beispielsweise Titandioxid, Zinkoxid, Eisenoxid, Aluminiumoixid, Ceroxid, Zirkoniumoxid, Silicate (Talk), Bariumsulfat und Zinkstearat. Die Partikel sollten dabei einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Eine relativ neue Klasse von Lichtschutzfiltern sind micronisierte organische Pigmente, wie beispielsweise 2,2'-Methylene-bis-{6-(2H-benzotriazol-2-yl)-4-(1,1,3,3-tetramethylbutyl)-phenol} mit einer Partikelgröße von < 200 nm, das beispielsweise als 50 %ige wässrige Dispersion erhältlich ist.

Neben den beiden vorgenannten Gruppen primärer Lichtschutzstoffe können auch sekundäre Lichtschutzmittel vom Typ der Antioxidantien eingesetzt werden, die die photochemische Reaktionskette unterbrechen, welche ausgelöst wird, wenn UV-Strahlung in die Haut eindringt. Typische Beispiele hierfür sind Aminosäuren (beispielsweise Glycin, Histidin, Tyrosin, Tryptophan) und deren Derivate, Imidazole (beispielsweise Urocaninsäure) und deren Derivate, Peptide wie D,L-Carnosin, D-Carnosin und deren Derivate (beispielsweise Anserin), Carotinoide, Carotine (beispielsweise α - Carotin, β-Carotin, Lycopin) und deren Derivate, Chlorogensäure und deren Derivate, Liponsäure und deren Derivate (beispielsweise Dihydroliponsäure), Aurothioglucose, Propylthiouracil und andere Thiole (beispielsweise Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl, Cholesteryl- und Glycerylester) sowie deren Salze, Dilaurylthiopropionat, Distearylthiopropionat, Thiodipropionsäure und deren Derivate (Ester, Ether, Peptide, Lipide, Nukleotide, Nukleoside und Salze) sowie Sulfoximinverbindungen (beispielsweise Buthioninsulfoximine, Homocysteinsulfoximin, Buthioninsulfone, Penta-, Hexa-, Heptathioninsulfoximin) in sehr geringen verträglichen Dosierungen (beispielsweise pmol bis µ mol/kg), ferner (Metall)-Chelatoren (beispielsweise α-Hydroxyfettsäuren, Palmitinsäure, Phytinsäure, Lactoferrin), α-Hydroxysäuren (beispielsweise Citronensäure, Milchsäure, Äpfelsäure), Huminsäure, Gallensäure, Gallenextrakte, Bilirubin, Biliverdin, EDTA, EGTA und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (beispielsweise Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihadroguajaretsäure, Trihydroxybutyrophenon, Harnsäure und deren Derivate, Mannose und deren Derivate, Superoxid-Dismutase, Zink und dessen Derivate (beispielsweise ZnO, ZnSO₄), Selen und dessen Derivate (beispielsweise Selen-methionin), Stilbene und deren Derivate (beispielsweise Stilbenoxid, trans-Stilbenoxid) und die erfindungsgemäß geeigneten Derivate (Salze, Ester, Ether, Zucker, Nucleotide, Peptide und Lipide) dieser genannten Wirkstoffe.

Als Konservierungsmittel eignen sich beispielsweise Phenoxyethanol, Formaldehydlösung, Parabene, Pentandiol oder Sorbinsäure.

Als Insekten-Repellentien kommen N,N-Diethyl-m-toluamid, 1,2-Pentandiol oder Insect Repellent 3535 in Frage, als Selbstbräuner eignet sich Dihydroxyaceton, als Parfümöle seien genannt Gemische aus natürlichen und synthetischen Riechstoffen. Natürliche Riechstoffe sind Extrakte von Blüten (Lilie, Lavendel, Rosen, Jasmin, Neroli, Ylang-Ylang), Stengeln und Blättern (Geranium, Patchouli, Petitgrain), Früchten (Anis, Koriander, Kümmel, Wacholder), Fruchtschalen (Bergamotte, Zitrone, Orangen), Wurzeln, (Macis, Angelica, Sellerie, Kardamon, Costus, Iris, Thymian), Nadeln und Zweigen (Fichte, Tanne, Kiefer, Latschen), Harzen und Balsamen (Galbanum, Elemi, Benzoe, Myrrhe, Olibanum, Opoponax). Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, Phenoxyethylisobutyrat, p-tert.-Butylcyclohexylacetat, Linalylacetat, Dimethylbenzylcarbinylacetat, Phenylethylacetat, Linalylbenzoat, Benzylformiat, Ethylmethyl-phenylglycinat, Allylcyclohexylpropionat, Styrallylpropionat und Benzylsalicylat. Zu den Ethern zählen beispielsweise Benzylethylether, zu den Aldehyden beispielsweise die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen beispielsweise die Jonone, α-Isomethylionon und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hautpsächlich die Terpene und Balsame. Bevorzugt werden jedoch Mischungen verschiedener Riechstoffe verwendet, die gemeinsam eine ansprechende Duftnote erzeugen. Auch ätherische Öle geringer Flüchtigkeit, die meist als Aromakomponenten verwendet werden, eignen sich als Parfümöle, beispielsweise Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labdanumöl und Lavandinöl. Vorzugsweise werden Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evernyl, Iraldein gamma, Phenylessigsäure, Geranylacetat, Benzylacetat, Rosenoxid, Romillat, Irotyl und Floramat allein oder in Mischungen, eingesetzt.

Als Farbstoffe können die für kosmetische Zwecke geeigneten und zugelassenen Substanzen verwendet werden, wie sie beispielsweise in der Publikation "Kosmetische Färbemittel" der Farbstoffkommission der Deutschen Forschungsgemeinschaft, Verlag Chemie, Weinheim, 1984, S. 81 - 106 zusammengestellt sind. Diese Farbstoffe werden üblicherweise in Konzentrationen von 0,001 bis 0,1 Gew.-%, bezogen auf die gesamte Mischung, eingesetzt.

Als Deodorantwirkstoffe kommen z. B. Geruchsüberdecker wie die gängigen Parfümbestandteile, Geruchsabsorber, beispielsweise die in der Offenlegungsschrift DE-A-40 09 347 beschriebenen Schichtsilikate, von diesen insbesondere Montmorillonit, Kaolinit, Illit, Beidellit, Nontronit, Saponit, Hectorit, Bentonit, Smectit, ferner beispielsweise Zinksalze der Ricinolsäure. Keimhemmende Mittel sind ebenfalls geeignet, in die erfindungsgemäßen Öl-in-Wasser-Emulsionen eingearbeitet zu werden. Vorteilhafte Substanzen sind zum Beispiel 2,4,4'-Trichlor-2'-hydroxydiphenylether (Irgasan), 1,6-Di-(4-chlorphenylbiguanido)-hexan (Chlorhexidin), 3,4,4'-Trichlorcarbanilid, quaternäre Ammoniumverbindungen, Nelkenöl, Minzöl, Thymianöl, Triethylcitrat, Farnesol (3,7,11-Trimethyl-2,6,10-dodecatrien-1-ol) sowie die in den Offenlegungsschriften DE-A-198 55 934, DE-A-37 40 186, DE-A-39 38 140, DE-A-42 04 321, DE-A-42 29 707, DE-A-42 29 737, DE-A-42 38 081, DE-A-43 09 372, DE-A-43 24 219 beschriebenen wirksamen Agenzien. Weitere übliche Antitranspirantwirkstoffe können ebenfalls vorteilhaft in den erfindungsgemäßen Zubereitungen verwendet werden, insbesondere Adstringentien, beispielsweise basische Aluminiumchloride wie Aluminiumchlorhydrat ("ACH") und Aluminium-Zirkonium-Glycine-Salze ("ZAG").

Als Wirkstoffe sind beispielsweise Tocopherol, Tocopherolacetat, Tocopherolpalmitat, Ascorbinsäure, Desoxyribonucleinsäure, Retinol, Bisabolol, Allantoin, Phytantriol, Panthenol, AHA-Säuren, Aminosäuren, Ceramide, Pseudoceramide, essentielle Öle, Pflanzenextrakte und Vitaminkomplexe geeignet.

### Ausführungsbeispiele:

In einer ersten Stufe wird beispielsweise Glutaminsäure bei 170 bis 190 °C geschmolzen, wobei das cyclische Amid, durch Abspaltung von Wasser die Pyroglutaminsäure, bildet. Man gibt dann Asparaginsäure und/oder andere Coaminosäuren zu und erhitzt die Schmelze weiterhin bei derselben Temperatur. Das entstehende Reaktionswasser wird kontinuierlich abdestilliert. Je länger die Dauer des Erhitzens und je höher die Temperatur, desto größer ist die Molekularmasse des entstehenden Peptids. Dieses Peptid kann auch in einem stufenlosen Verfahren hergestellt werden, wobei Coaminosäure, Glutaminsäure und gegebenfalls weitere Aminosäuren gleichzeitig bei derselben Temperatur erwärmt und polymerisiert werden.

Im nächsten Schritt werden Amine zu dem Peptid zugegeben und bei 170 °bis 190 °C weiterreagiert. Die entstandene Schmelze wird entweder ausgegossen und nach Abkühlen zerkleinert, oder direkt im Anschluss mit wässrigen Basen, z.B. wässrige Natronlauge, behandelt. Die so entstandene Suspension kann entweder direkt weiterverwendet werden oder isoliert werden.

In einer Verfahrensvariante wird nach der Glutaminsäure, das Amin zugegeben und erst im darauffolgenden Schritt Asparaginsäure oder/und andere Coaminosäuren dazugegeben.

In einer weiteren Verfahrensvariante können alle Komponenten zusammengemischt und unter den angegebenen Bedingungen zur Reaktion gebracht werden.

### Herstellungsbeispiele 1 bis 8:

Beispiele erfindungsgemäß verwendeter hydrophob modifizierter Polyglutaminsäurederivate sind im folgenden aufgeführt:

### Beispiel 1:

In einem 2-l-Planschliffkolben, ausgestattet mit Ölbad, Rührer, Thermometer und Destillationsbrücke, werden 221 g Glutaminsäure und 200 g Asparaginsäure unter geringem Stickstoffstrom vorgelegt und auf 180 °C erwärmt. Das entstehende Reaktionswasser wird über die Destillationsbrücke abdestilliert und kondensiert. Nachdem werden 54 g Wasser abdestilliert. Anschließend gibt man während einer halbe Stunde 105 g Stearylamin (Armeen 18 D von Akzo Nobel) langsam zu und rührt noch eine halbe Stunde bei derselben Temperatur. Bei 140 °C wird solange eine 25 %ige Natronlauge zugegeben bis ein pH-Wert von 5 eingestellt ist. Das Produkt kann durch Zugabe von 1 g Aminoiminomethansulfinsäure bei 80 °C während einer Stunde gebleicht werden. Anschließend werden 10 g 30 %ige Wasserstoffperoxidlösung zugegeben und nochmals eine Stunde bei 80 °C gerührt.

Das Produkt wird am Rotationsverdampfer eingeengt. Die Restmengen der Aminosäuren können über HPLC bestimmt werden. Man erhält eine hellbeige 50 %ige Suspension mit ca. 5 % freie Asparaginsäure und 18 % pyro-Glutaminsäure.

### Beispiel 2:

In einem 2-l-Planschliffkolben, ausgestattet mit Ölbad, Rührer, Thermometer und Destillationsbrücke, werden 80 g Glutaminsäure, 320 g Asparaginsäure und 100 g Stearylamin (Armeen 18 D von Akzo Nobel) unter geringem Stickstoffstrom vorgelegt und auf 190° C erwärmt. Nach 1,5 h wird die Temperatur auf 220 °C erhöht und weitere 2,5 h gerührt. Das entstehende Reaktionswasser (53 g) wird über die Destillationsbrücke abdestilliert und kondensiert. Bei 140 °C werden solange eine 25 %ige Natronlauge und Wasser zugegeben bis ein pH-Wert von 7 eingestellt ist. Das Produkt kann durch Zugabe von 1 g Aminoiminomethansulfinsäure bei 80 °C innerhalb einer Stunde gebleicht werden. Anschließend werden 10 g 30 %ige Wasserstoffperoxidlösung zugegeben und nochmals eine Stunde bei 80 °C gerührt. Das Produkt wird am Rotationsverdampfer eingeengt. Die Restmengen der Aminosäuren können über HPLC bestimmt werden. Man erhält eine hellbeige 29 %ige Suspension mit ca. 14 % freie Asparaginsäure, 2 % Glutaminsäure und 3 % pyro-Glutaminsäure.

### Beispiel 3:

In einem 2-l-Planschliffkolben, ausgestattet mit Ölbad, Rührer, Thermometer und Destillationsbrücke werden 250 g Glutaminsäure unter geringem Stickstoffstrom vorgelegt und auf 180 °C erwärmt. Das entstehende Reaktionswasser wird über die Destillationsbrücke abdestilliert und kondensiert (30 g). Nach 1,5 h werden 119 g Stearylamin zugegeben und 0,5 h bei 180 °C gerührt. Danach wird 226 g Asparaginsäure zugegeben und nach 1 h sind 26 g Wasser abdestilliert. Bei 150°C wird solange eine 25 %ige Natronlauge zugegeben bis ein pH-Wert von 5 eingestellt ist. Nach Einengen am Rotationsverdampfer erhält man ein hellgelbes Pulver. Die Restmengen der Aminosäuren können über HPLC bestimmt werden. Man erhält ein modifiziertes Peptid mit 21 % freier Asparaginsäure, 18 % pyro-Glutaminsäure und 4 % freie Glutaminsäure.

### Beispiel 4:

In einem 2-l-Planschliffkolben, ausgestattet mit Ölbad, Rührer, Thermometer und Destillationsbrücke, werden 500 g Glutaminsäure und 45 g Asparaginsäure unter geringem Stickstoffstrom vorgelegt und auf 220 °C erwärmt. Das entstehende Reaktionswasser wird über die Destillationsbrücke abdestilliert und kondensiert.(67 g). Anschließend gibt man innerhalb einer halbe Stunde 105 g Stearylamin (Armeen 18 D von Akzo Nobel) langsam zu. Bei 140 °C wird solange eine 25 %ige Natronlauge zugegeben bis ein pH-Wert von 7 eingestellt ist.

Das Produkt wird am Rotationsverdampfer eingeengt. Die Restmengen der Aminosäuren können über HPLC bestimmt werden. Man erhält ein gelbes Pulver mit ca. 2 % freier Glutaminsäure und 50 % pyro-Glutaminsäure.

### Beispiel 5:

In einem 2-l-Planschliffkolben, ausgestattet mit Ölbad, Rührer, Thermometer und Destillationsbrücke, werden 74 g Glutaminsäure und 67 g Asparaginsäure unter geringem Stickstoffstrom vorgelegt und auf 180 °C erwärmt. Das entstehende Reaktionswasser wird über die Destillationsbrücke abdestilliert und kondensiert (18 g). Anschließend gibt man innerhalb einer halbe Stunde 270 g Stearylamin (Armeen 18 D von Akzo Nobel) langsam zu und rührt noch 0,5 Stunden bei 150 °C. Bei 140 °C wird solange eine 25 %ige Natronlauge zugegeben bis ein pH-Wert von 7 eingestellt ist. Das Produkt wird am Rotationsverdampfer eingeengt. Die Restmengen der Aminosäuren können über HPLC bestimmt werden. Man erhält ein modifiziertes Peptid mit ca. 2 % freier Asparaginsäure und 5 % pyro-Glutaminsäure.

### Beispiel 6:

In einem 2-l-Planschliffkolben, ausgestattet mit Ölbad, Rührer, Thermometer und Destillationsbrücke, werden 221 g Glutaminsäure und 200 g Asparaginsäure unter geringem Stickstoffstrom vorgelegt und auf 180 °C erwärmt. Das entstehende Reaktionswasser wird über die Destillationsbrücke abdestilliert und kondensiert.

Nachdem 54 g Wasser abdestilliert werden, gibt man während einer Stunde 307 g Dimethylaminopropylamin langsam zu und rührt noch eine Stunde bei derselben Temperatur. Man erhält eine braune Lösung, die nach dem Abkühlen hochviskos und klebrig ist.

### Beispiel 7:

In einem 2-l-Planschliffkolben, ausgestattet mit Ölbad, Rührer, Thermometer und Destillationsbrücke, werden 200 g Glutaminsäure und 200 g Asparaginsäure unter geringem Stickstoffstrom vorgelegt und auf 180 °C erwärmt. Das entstehende Reaktionswasser wird über die Destillationsbrücke abdestilliert und kondensiert.

Nachdem 52 g Wasser abdestilliert werden, gibt man innerhalb einer halbe Stunde 100 g Stearylamin (Armeen 18 D von Akzo Nobel) langsam zu und rührt noch eine halbe Stunde bei derselben Temperatur. 100 g Glycin werden anschließend innerhalb einer halben Stunde zugegeben und wiederum eine halbe Stunde bei gleichbleibender Temperatur gerührt. Bei 140 °C wird solange eine 25 %ige Natronlauge zugegeben bis ein pH-Wert von 7 eingestellt ist. Man erhält eine braune Suspension mit ca. 54 % Festkörpergehalt.

### Beispiel 8:

In einem 2-l-Planschliffkolben, ausgestattet mit Ölbad, Rührer, Thermometer und Destillationsbrücke, werden 200 g Glutaminsäure und 181 g Asparaginsäure unter geringem Stickstoffstrom vorgelegt und auf 180 °C erwärmt. Das entstehende Reaktionswasser wird über die Destillationsbrücke abdestilliert und kondensiert.

Nachdem 48 g Wasser abdestilliert werden, wird eine halbe Stunde bei derselben Temperatur gerührt. Anschließend gibt man während einer halbe Stunde 95 g Polyoxyalkylenamin (Jeffamin M-600 von Huntsman) langsam zu und rührt noch eine halbe Stunde bei derselben Temperatur. Bei 140 °C wird solange eine 25 %ige Natronlauge zugegeben bis ein pH-Wert von 5 eingestellt ist. Das Produkt kann durch Zugabe von 1 g Aminoiminomethansulfinsäure bei 80 °C innerhalb einer Stunde gebleicht werden. Anschließend werden 10 g 30 %ige Wasserstoffperoxidlösung zugegeben und nochmals eine Stunde bei 80 °C gerührt.

Das Produkt wird am Rotationsverdampfer eingeengt. Die Restmengen der Aminosäuren können über HPLC bestimmt werden. Man erhält eine 53 %ige klare hellbraune Lösung mit ca. 5 % freier Asparaginsäure und 22 % pyro-Glutaminsäure.

Beispiele erfindungsgemäßer Öl-in-Wasser-Emulsionen sind im folgenden aufgeführt:

**Tabelle 1:**

| | | |
|---|---|---|
| A | Polyglutaminsäurederivat Herstellungsbeispiel 1 | 0,5 % |
| | TEGIN M (Glyceryl Stearate) | 3,0 % |
| | TEGO Alkanol 1618 (Cetearyl Alcohol) | 2,0 % |
| | Capryl/Caprin-Triglycerid | 9,0 % |
| | Ethylhexyl-Stearat | 4,0 % |
| | Mineral-Öl (30 mPas) | 5,0 % |
| | Tocopheryl-Acetat | 1,0 % |
| B | Glycerin | 2,0 % |
| | Panthenol | 1,0 % |
| | Allantoin | 0,1 % |
| | Wasser | 70,2 % |
| C | TEGO Carbomer 134 (Carbomer) | 0,15 % |
| | Ethylhexyl-Stearat | 1,6 % |
| D | Natriumhydroxid (10 % in Wasser) | 0,45 % |
| | Konservierungsstoff, Parfum | q. s. |

**Tabelle 2:**

| | | |
|---|---|---|
| A | Polyglutaminsäurederivat Herstellungsbeispiel 6 | 1,0 % |
| | TEGIN M (Glyceryl Stearate) | 4,0 % |
| | TEGO Alkanol 1618 (Cetearyl Alcohol) | 2,0 % |
| | Capryl/Caprin-Triglycerid | 9,0 % |
| | Ethylhexyl-Stearat | 4,0 % |
| | Mineral-Öl (30 mPas) | 5,0 % |
| B | Glycerin | 3,0 % |
| | Wasser | 70,8 % |
| C | TEGO Carbomer 141 (Carbomer) | 0,15 % |
| | Ethylhexyl-Stearat | 0,6 % |
| D | Natriumhydroxid (10 % in Wasser) | 0,45 % |
| | Konservierungsstoff, Parfum | q. s. |

**Tabelle 3:**

| | | |
|---|---|---|
| A | Polyglutaminsäurederivat Herstellungsbeispiel 8 | 1,0 % |
| | PEG-100 Stearat | 1,0 % |
| | Stearyl-Alkohol | 2,0 % |
| | Stearinsäure | 2,0 % |
| | Capryl/Caprinsäure-Triglycerid | 7,0 % |
| | Ethylhexyl-Stearat | 6,2 % |
| | Tocopheryl-Acetat | 0,3 % |
| B | Glycerin | 2,0 % |
| | Panthenol | 0,5 % |
| | Allantoin | 0,2 % |
| | Wasser | 77,05 % |
| C | TEGO Carbomer 134 (Carbomer) | 0,1 % |
| | Mineral-Öl (30 mPas) | 0,4 % |
| D | Natriumhydroxid (10 % in Wasser) | 0,25 % |
| | Konservierungsstoff, Parfum | q. s. |

Bei den hier beschriebenen Beispielen erfindungsgemäßer Öl-in-Wasser-Emulsionen handelt es sich durchweg um feindisperse Emulsionen mit brillantem Aussehen und einem angenehmen Hautgefühl, deren pH-Wert bei ca. 5,5 liegt und somit dem pH-Wert des natürlichen Säureschutzmantels der Haut entspricht. Sie zeichnen sich ferner durch eine sehr gute Langzeit- sowie Kälte- und Wärmestabilität aus.

## Patentansprüche

1. Kosmetische oder pharmazeutische Öl-in-Wasser-Emulsionen, die ein oder mehrere hydrophob modifizierte copolymere Polyglutaminsäurederivate und weitere Hilfsund Zusatzstoffe enthalten, **dadurch gekennzeichnet, dass** als copolymere Polyglutaminsäurederivate Verbindungen verwendet werden, welche durch gleichzeitige oder stufenweise Umsetzung von Glutaminsäure und mindestens einer weiteren α-Aminosäure und/oder deren Derivaten und Aminen in Abwesenheit von Lösungsmitteln und Katalysatoren hergestellt werden.

2. Kosmetische oder pharmazeutische Öl-in-Wasser-Emulsionen, **dadurch gekennzeichnet, dass** sie enthalten
(a) ein oder mehrere hydrophob modifizierte copolymere Polyglutaminsäurederivate hergestellt gemäß Anspruch 1 und
(b) ein oder mehrere polare Wachse ausgewählt aus der Gruppe bestehend aus Fettalkoholen mit 12 bis 22 Kohlenstoffatomen, bestehend aus Fettsäuren mit 12 bis 22 Kohlenstoffatomen und/oder bestehend aus Glycerin- oder Polyglycerinpartialestern von Fettsäuren mit 12 bis 22 Kohlenstoffatomen und
(c) ein oder mehrere kosmetische Öle.

3. Kosmetische oder pharmazeutische Öl-in-Wasser-Emulsionen gemäß Anspruch 2, **dadurch gekennzeichnet, dass** der Anteil der Komponente (a) zwischen 0,1 und 2,0 %, der Anteil der Komponente (b) 0,5 bis 8,0 % und der Anteil der Komponente (c) 1,0 bis 60 % an der Gesamtemulsion beträgt.

4. Kosmetische oder pharmazeutische Öl-in-Wasser-Emulsionen gemäß Anspruch 3, **dadurch gekennzeichnet, dass** sie zusätzlich Stabilisatoren, ausgewählt aus der Gruppe bestehend aus synthetischen oder natürlichen Hydrocolloiden, enthalten.

5. Kosmetische oder pharmazeutische Öl-in-Wasser-Emulsionen nach einem der vorhergehenden Ansprüche **dadurch gekennzeichnet, dass** die Emulsionen weitere für kosmetische Emulsionen übliche Hilfs- und Zusatzstoffe enthalten, die ausgewählt sind aus der Gruppe bestehend aus Emulgatoren, UV-Lichtschutzfiltern, Antitranspirantien, Deodorantien, Antioxidantien, Konservierungsmitteln, Insekten-Repellentien, Selbstbräunern, Parfumölen, Farbstoffen und Wirkstoffen.

## Claims

1. Cosmetic or pharmaceutical oil-in-water emulsions which comprise one or more hydrophobically modified copolymeric polyglutamic acid derivatives and further auxiliaries and additives, **characterized in that** the copolymeric polyglutamic acid derivatives used are compounds which are prepared by simultaneous or stepwise reaction of glutamic acid and at least one further α-amino acid and/or derivatives thereof and amines in the absence of solvents and catalysts.

2. Cosmetic or pharmaceutical oil-in-water emulsions, **characterized in that** they comprise
(a) one or more hydrophobically modified copolymeric polyglutamic acid derivatives prepared as in claim 1 and
(b) one or more polar waxes chosen from the group consisting of fatty alcohols having 12 to 22 carbon atoms, consisting of fatty acids having 12 to 22 carbon atoms and/or consisting of glycerol or polyglycerol partial esters of fatty acids having 12 to 22 carbon atoms and
(c) one or more cosmetic oils.

3. Cosmetic or pharmaceutical oil-in-water emulsions according to Claim 2, **characterized in that** the proportion of component (a) is between 0.1 and 2.0%, the proportion of component (b) is 0.5 to 8.0% and the proportion of component (c) is 1.0 to 60% of the overall emulsion.

4. Cosmetic or pharmaceutical oil-in-water emulsions according to Claim 3, **characterized in that** it additionally comprises stabilizers chosen from the group consisting of synthetic or natural hydrocolloids.

5. Cosmetic or pharmaceutical oil-in-water emulsions according to any of the preceding claims, **characterized in that** the emulsions comprise further auxiliaries and additives customary for cosmetic emulsions which are chosen from the group consisting of emulsifiers, UV light protection filters, antiperspirants, deodorants, antioxidants, preservatives, insect repellents, self-tanning agents, perfume oils, dyes and active ingredients.

## Revendications

1. Emulsions huile dans eau cosmétiques ou pharmaceutiques qui contiennent un ou plusieurs dérivés de poly(acide glutamique) copolymères modifiés de manière hydrophobe et d'autres adjuvants et additifs, **caractérisées en ce qu'**on utilise comme dérivés de poly(acide glutamique) copolymères des composés qui sont préparés par transformation simultanée ou par étapes d'acide glutamique et d'au moins un autre acide α-aminé et/ou ses dérivés et des amines en l'absence de solvants et de catalyseurs.

2. Emulsions huile dans eau cosmétiques ou pharmaceutiques, **caractérisées en ce qu'**elles contiennent
(a) un ou plusieurs dérivés de poly(acide glutamique) copolymères modifiés de manière hydrophobe, préparés selon la revendication 1 et
(b) une ou plusieurs cires polaires, choisies dans le groupe constitué par les alcools gras comprenant 12 à 22 atomes de carbone, constitué par les acides gras comprenant 12 à 22 atomes de carbone et/ou constitué par les esters partiels de glycérol ou de polyglycérol d'acides gras comprenant 12 à 22 atomes de carbone et
(c) une ou plusieurs huiles cosmétiques.

3. Emulsions huile dans eau cosmétiques ou pharmaceutiques selon la revendication 2, **caractérisées en ce que** la proportion du composant (a) est comprise entre 0,1 et 2,0%, la proportion du composant (b) est de 0,5 à 8,0% en poids et la proportion du composant (c) est de 1,0 à 60% par rapport à l'émulsion totale.

4. Emulsions huile dans eau cosmétiques ou pharmaceutiques selon la revendication 3, **caractérisées en ce qu'**elles contiennent en outre des stabilisants, choisis dans le groupe constitué par les hydrocolloïdes synthétiques ou naturels.

5. Emulsions huile dans eau cosmétiques ou pharmaceutiques selon l'une quelconque des revendications précédentes, **caractérisées en ce que** les émulsions contiennent d'autres adjuvants et additifs usuels pour des émulsions cosmétiques, choisis dans le groupe constitué par les émulsifiants, les filtres de protection contre la lumière UV, les antiperspirants, les déodorants, les antioxydants, les agents de conservation, les répulsifs pour insectes, les autobronzants, les huiles parfumées, les colorants et les substances actives.
